# EUROPEAN PATENT SPECIFICATION

(11) **EP 1 200 557 B1**
(45) Date of publication and mention of the grant of the patent: **26.04.2006**
(21) Application number: 00952574.2
(22) Date of filing: 07.08.2000
(51) Int. Cl.: C12N 5/04, C12N 15/05, C12N 15/09, C12N 15/81, C12N 15/82, C12N 15/87, C12N 15/90, A01H 1/00, A01H 5/00

(54) **METHOD OF MAKING PLANT ARTIFICIAL CHROMOSOMES**
VERFAHREN ZUR HERSTELLUNG KÜNSTLICHER PFLANZENCHROMOSOMEN
PROCEDE DE FABRICATION DE CHROMOSOMES ARTIFICIELS DE PLANTES

(30) Priority: 05.08.1999 US 147445 P
(43) Date of publication of application: 02.05.2002
(73) Proprietor: Icon Genetics, Inc., Princeton, NJ 08542 (US)
(72) Inventor: KLIMYUK, Victor, Norwich NIR4 7EG (GB); KUCHUK, Nikolay V., Kiev 252179 (UA)
(74) Representative: Bergstrand, Mikael Gudmundsson
(86) International application number: PCT/US2000/021461
(87) International publication number: WO 2001/011020

(56) References cited:
- WO-A1-98/55637
- US-A- 5 270 201
- DE KOCHKO ALEXANDRE: "De l'utilisation des chromosomes artificiels de plantes comme outil pour la conservation et l'exploitation des ressources genetiques vegetales." CAHIERS AGRICULTURES, vol. 9, no. 4, 2000, pages 287-292, XP001121303 ISSN: 1166-7699
- SUZUKI ET AL.: 'Direct cloning of the brassica S locus by using a P1-derived artificial chromosome (PAC) vector' GENE vol. 199, 1997, pages 133 - 137, XP002933436
- QUONZHOU ET AL.: 'Cloning and stable maintenance of DNA fragments over 300kb in escherichia coli with conventional plasmid-based vectors' NUCL. ACIDS RES. vol. 26, no. 21, 1998, pages 4901 - 4909, XP002933437

## Description

### TECHNICAL FIELD

The present invention relates to the field of molecular genetics and particularly to the production of artificial chromosomes such as plant artificial chromosomes.

### BACKGROUND OF THE INVENTION

Several methods have been developed for the introduction and expression of genes in plant and animal cells and particularly plant cells. These methods have a number of limitations, especially considering the complexity of the breeding process required for the introgression of more than a few genes into elite lines and obtaining stable, predictable expression of the individual genes. The current methods for plant transformation are limited to the introduction of small segments of DNA, generally sufficient for the expression of two to three genes. With the rapid increase in rate of sequencing and the discovery of new genes for modifying agronomic traits and for directing plants to synthesize material from entirely new pathways, this limitation will be severely limiting. In addition, the current method of randomly introducing genes into the genome of the recipient plant leads to extensive linkage drag, potential for disruption of important genes and confounding the production of elite lines.

The artificial chromosome is a linear piece of DNA that contains all the necessary elements for stable replication and segregation. Artificial chromosomes have been described for yeast (Burke *et al.,* Science 236:806-812, (1987)), bacteria (O'Connor *et al.,* Science *244(4910):*1307-1312 (1989), Shizuya *et al.,* Proc. Natl. Acad. Sci. USA *89*(18):8794-8797 (1992), Hosoda *et al.,* Nucleic Acids Res. *18*(*13*):3863-3869 (1990)), and more recently for animals (Harrington *et al.,* Nature Genetics 15:345-355 (1998); Grimes and Cooke, Human Molecular Genetics *7(10)*:1635-1640 (1998); and *Ikeno et al.,* Nature Biotechnology 16:431-439 (1998)). In these cases, the chromosomes were produced by identifying the required elements and then manipulating them to build a chromosome, or via *in vivo* and *in vitro* manipulations involving isolation of one or more chromosomal elements.

U.S. Patent 5,270,201 describes telomeric sequences from *Arabidopsis* and use of those sequences to construct a plant artificial chromosome. The patent disclosure relates to a recombinant DNA molecule that contains the telomere and optionally the centromere of a higher eukaryote. To provide a functional artificial chromosome in accordance with the teachings of the patent, the functional elements of a chromosome must be assembled and transformed into a plant cell. The element exemplified in the patent, the telomere, is the simplest one of the necessary pieces. Presently however, a plant centromere is known to be a highly complex structure of at least 360,000 base pairs. More recently, PCT application (WO 98/55637) describes the identification and cloning of functional plant centromeres based on *Arabidopsis.*

Hence, there is a need in the agricultural biotechnology arts for methods of producing plant artificial chromosomes that entail less complex genetic manipulation and assembly of individual chromosomal elements.

### SUMMARY OF THE INVENTION

Applicants have invented methods of producing plant artificial chromosomes. The method is generally applicable to any plant species of interest including dicots and monocots. The methods take advantage of the natural ability of a plant cell to repair damage done to its chromosomes. Rather than synthesize artificial chromosomes from known functional chromosomal elements such as centromeres, telomeres and autonomously replicating sequences, the present invention utilizes the normal metabolic functions of a plant cell to perform all necessary processes to create functional minichromosomes.

A first aspect of the present invention is directed to a method of making a plant artificial chromosome. It entails the following:
a) preparing recombinant protoplasts of a first plant species containing an exogenous or non-native nucleic acid (e.g., DNA) of interest; b) producing chromosome fragments of chromosomes contained in the recombinant protoplasts; c) fusing the recombinant protoplasts of (b) with protoplasts of a second plant species to produce fused protoplasts, wherein the first and second plant species may be the same or different; and d) identifying fused protoplasts of c) or cells derived from the fused protoplasts of (c) that contain chromosome fragments that exhibit normal plant chromosomal properties.

In another embodiment, the plant artificial chromosome is made by the following procedure:
a) producing transformed plants of a first plant species containing an exogenous nucleic acid; b) producing chromosome fragments of chromosomes of the first plant species; c) crossing the first plant species containing the chromosome fragments with a second plant species to produce hybrid plant species wherein the first and second plant species may be the same or different; and d) identifying hybrid plant species of c) or cells or protoplasts thereof containing at least one chromosome fragment that exhibits normal plant chromosomal functions.

In preferred embodiments, chromosome fragments are produced by irradiating the protoplasts or treating with a chemical agent. Fused protoplasts or cells derived from the fused protoplasts that contain chromosome fragments exhibiting normal plant chromosomal properties are identified by pulsed field gel electrophoresis. The first and second plants may be members of the same species or family, or they may be unrelated. The methods are applicable to all plants - - monocots and dicots alike.

In other preferred embodiments, the exogenous DNA contains at least one functional site such as a recombination site, a restriction site and/or a coding region. Selectable marker genes are generally included as a part of the coding region. Yeast chromosomal elements, e.g., yeast artificial chromosomes, are preferred. In these cases, wherein the coding region contains a centromeric sequence functional in a yeast cell, the method of the present invention produces a chromosomal fragment containing another centromeric sequence functional in a plant cell, as well as the centromeric sequence functional in a yeast cell. These DNAs are reconstructed into a recombinant or shuttle vector and are used to produce transformed or recombinant plant or yeast cells. More generally, however, the plant artificial chromosomes (PACs) produced by the presently disclosed methods, and constructs and cells transformed with the PACs, are also provided.

Another aspect of the present invention is directed to whole plants produced by the methods, such as by regenerating the plants from the fused protoplasts. Isolated plant cells and plant cell and protoplast cultures are also disclosed.

A further aspect of the present invention is directed to a method of making a transgenic plant. This method entails a) preparing recombinant protoplasts of a first plant species containing an exogenous nucleic acid; b) producing chromosome fragments of chromosomes contained in the recombinant protoplasts; c) fusing the recombinant protoplasts of (b) with protoplasts of a second plant species to produce fused protoplasts, wherein the first and second plant species may be the same or different; d) identifying fused protoplasts of c) or cells derived from the fused protoplasts of (c) that contain chromosome fragments that exhibit normal plant chromosomal properties; and e) regenerating a whole plant from the protoplasts or cells identified in d) that contain the chromosome fragments exhibiting normal plant chromosomal properties. Seed derived from the transgenic plants is also provided.

### BRIEF DESCRIPTION OF THE DRAWINGS

Fig.1 is a schematic presentation of the *in vivo* generation of plant artificial chromosomes in accordance with the present invention;
Fig. 2 is a photograph illustrating metaphase chromosomes of "gamma"-hybrid between *Nicotiana plunibaginifolia* and *Atropa belladonna,* wherein (N) represents Nicotiana chromosomes; (A) represents *Atropa* chromosomes; (m) represents minichromosomes produced by "gamma"-treatment of *Atropa* protoplast; and (r) represents reconstructed chromosome;
Fig. 3 is a plasmid map of plasmid pYAC-GN; and
Figs. 4A and 4B are plasmid maps of pIG461 and pIC462.

### DETAILED DESCRIPTION OF THE INVENTION

The invention relates to the production of artificial chromosomes and the preparation of cell lines containing functional artificial chromosomes. Also provided are methods for introducing DNA into the artificial chromosome by targeted integration and delivery of the artificial chromosome into recipient cells. Cells that can harbor and that can be used in the manipulation of the artificial chromosome include yeast and cells from monocotyledonous and dicotyledonous plants, and the cell cultures and regenerated plants from those cells.

The telomere is a stretch of DNA at the ends of chromosomes that are required for the complete replication of the chromosomal ends. A eukaryotic chromosome would shorten after each round of replication except for the presence of the telomere at each end. The telomere has a characteristic DNA sequence that is replicated differently from the bulk of the chromosome. The telomere serves as primer for the completion of the lagging strand as the chromosome replicates.

Isolation of the first eukaryotic telomere was accomplished in 1988 from *Arabidopsis.* Studies of the *Arabidopsis* telomeres showed that the structure of this DNA is very similar to that seen in lower eukaryotes. Telomere structure appears to be well conserved throughout the angiosperms. The telomeres of the monocot maize are varied in size and cross-hybridize with the telomeric sequence of *Arabidopsis.* The conservation of telomere structure and sequence is also seen into the animal kingdom.

The centromere is required for the accurate segregation of the sister chromatids after replication. The centromere consists of a sequence that is distinct from the rest of the chromosome. Kinetochores, which form at the centromere, attach to the spindle during mitosis and meiosis and are responsible for separation of the chromosomes. Centromeres are typically composed of large arrays of tandemly repeated DNA families. See, Clarke, Curr. Opin. Gen. Dev. *8*:212-218 (1998) and Pidoux, et al., Curr. Opin. Cell Biol. *12*:308-319 (2000).

The DNA fragments conferring the function of autonomously replicating sequences (ARS) have been isolated and characterized from many plant species. See, Berlani *et al.,* Plant Mol. Biol., *11*:161-162 (1988); Hernandes *et al.,* Plant Mol. Biol., *10*:413-422 (1988); Berlani *et al.,* Plant Mol. Biol., *11*:173-182 (1988); and Eckdahl *et al.,* Plant Mol. Biol., *12*:507-516 (1989). ARS elements from genomes of higher plants have structural and sequence features in common with ARS elements from yeast and higher animals (Eckdahl *et al.,* Plant Mol. Biol., *12*:507-516 (1989)). The plant ARS elements are capable of conferring autonomous replicating ability to plasmids in *Saccharomyces cerevisiae.* Study of maize nuclear DNA sequences capable of promoting the autonomous replication of plasmids in yeast showed that they represent two families of highly repeated sequences within the maize genome. Those sequences have characteristic genomic hybridization patterns. There was typically only one copy of an ARS-homologous sequence on each 12-15 kb of genomic fragment (Berlani *et al.,* Plant Mol. Biol., *11*:161-162 (1988)).

In accordance with one embodiment of the presently disclosed invention, a plant artificial chromosome (PAC) is produced first by introducing the exogenous DNA *e.g*., one or more gene(s) of interest including or associated with a selectable marker gene, into the desired plant species (*e.g*., maize). The selection of vector for use will depend upon the preferred transformation technique and the target species for transformation. For certain target species, different antibiotic or herbicide selection markers may be preferred. Selection markers used routinely in transformations include the *nptII* gene which confers resistance to kanamycin (Messing, *et al.,* Gene *19*:259-268 (1982); Bevan, *et al.,* Nature *304*:184-187 (1983)), the *bar* gene which confers resistance to the herbicide phosphinothricin (White, *et al.,* Nucl. Acids Res. *18:* 1062 (1990); Spencer, *et al.,* Theor. Appl. Genet. *79* :625-631 (1990)), the *hph* gene which confers resistance to the antibiotic hygromycin (Blochinger, *et al.,* Mol. Cell Biol. *4*:2929-2931)), and the *dhfr* gene, which confers resistance to methotrexate. Vectors suitable for *Agrobacterium* transformation typically carry at least one T-DNA border sequence. These include vectors such as pBIN19 and pCIB200 (EP 0 332 104).

Stable transformed cell lines are then selected which express the exogenous or non-native nucleic acid. The cells may be either from a whole regenerated plant following transformation and selection, or the cells may be obtained from suspension culture following transformation and selection. Methods of transforming plant cells or protoplasts to integrate exogenous DNA in the plant chromosome may be performed in accordance with standard procedures. Choosing a specific technique will depend primarily on whether the plant is a monocot or dicot.

Transformation techniques for dicotyledons are well known in the art and include *Agrobacterium-*based techniques and techniques that do not require *Agrobacterium* Non*-Agrobacterium* techniques involve the uptake of exogenous genetic material directly by protoplasts or cells. These techniques include PEG or electroporation mediated uptake, particle bombardment-mediated delivery and microinjection. Examples of these techniques are described in Paszkowski *et al.,* EMBO J., *3*:2717-2722 (1984), Potrykus *et al.,* Mol. Gen. Genet. *199*:169-177 (1985), Reich *et al.,* Biotechnology *4*:1001-1004 (1986), and Klein *et al.,* Nature, 327:70-73 (1987). In each case, the transformed cells are regenerated to whole plants using standard techniques.

Preferred transformation techniques for monocots include direct gene transfer into protoplasts using PEG or electroporation techniques and particle bombardment into callus tissue. Transformation can be undertaken with a single DNA species or multiple DNA species (*i.e*., co-transformation) and both these techniques are suitable for use with this invention. Co-transformation may have the advantage of avoiding complex vector construction and of generating transgenic plants with unlinked loci for the gene of interest and the selectable marker, enabling the removal of the selectable marker in subsequent generations, should this be regarded desirable. However, a disadvantage of the use of co-transformation is the less than 100% frequency with which separate DNA species are integrated into the genome (Schocher *et al.,* Biotechnology *4*:1093-1096 (1986)). Generation and rescue of minichromosomes

Protoplasts are derived from the transformed cells in accordance with standard techniques. In one preferred embodiment, fragments of the plant chromosomes (also referred to as "minichromosomes") are produced by irradiating the protoplasts. The irradiation renders the protoplasts non-viable. High doses of gamma radiation (*e.g*., 1000 Gy from a Cobalt-60 source) are particularly suitable. There are other methods for fragmenting chromosomal DNA. For example, the protoplasts or cells may be treated or otherwise contacted with a chemical agent. Examples of such agents include calicheamicin, esperamicin, dynemicin and neocarzinostatin. These agents are believed to mediate chromosomal cleavage via transient di-radical intermediates. See, Lee, *et al.,* J. Antibiot. *42*:1970 (1989); Lee, *et al.,* J. Am. Chem. Soc. *114*:985 (1992); and *Golik, et al.,* J. Am. Chem. Soc. *109*:3461 (1987).

The treated protoplasts are fused with non-transformed or normal (*e.g.*, undamaged) protoplasts derived from cells of the same plant species or a different but related *(i.e.,* the same family of) plant species, in accordance with standard techniques. The purpose of fusing the treated protoplasts with non-transformed protoplasts is to revive the transformed protoplasts from the effects of the chromosomal disruption e.g., caused by irradiation or chemical treatment.

The fused protoplasts are cultured on selective media to allow whole plant cells to form and to identify plant cells that contain and express the gene(s) of interest. These cells are screened by any suitable method (FISH, GISH, PFGE, Southern blot, etc.) to identify lines that have the gene of interest on a minichromosome that otherwise exhibits normal chromosomal activities. By "normal chromosomal activities", it is meant that the microchromosomes, artificial chromosome or chromosome fragments contain a centromere, telomeric and ARS sequences and are stable through normal cellular events such as meiosis and mitosis. That is, they are capable of independent replication and transmission through subsequent cell divisions.

After analyzing the stability of the desired microchromosome, it can be used as an artificial chromosome. This cell line or plant can be treated again as described above to select even smaller microchromosomes or PACs. The plant artificial chromosomes can be easily moved from one plant species to another by different means including methods based on unstable hybrid formation.

In a more preferred embodiment of this invention, the initial transformants are screened by appropriate means (*e.g.*, FISH, RAPD, PFGE and linkage analysis) to identify cell lines that have the gene(s) of interest located near the centromeric region of a chromosome. This preferred embodiment not only provides a higher probability of recovering microchromosomes that contain the desired gene(s) but results in the selection of the shortest fragments that contain the exogenous DNA and exhibit normal chromosomal activities. In general, the size of the minichromosomes lies in the range of from about 3 to about 4 Mb.p.

Chromosome fragments that exhibit normal chromosomal activity and contain the exogenous DNA can be isolated from the protoplasts and then further manipulated on a genetic level to incorporate additional exogenous DNA material of interest, and then transformed into a plant of interest. The isolated PACs can be introduced into the selected plant species in accordance with standard techniques such as electroporation or protoplasts or PEG-mediated transformation. In other embodiments, however, the fragments are not isolated; rather, are moved from plant cell to plant cell by successive fusion with protoplasts of the selected plant species. In these embodiments, the plant species containing the chromosome fragments (the "donor") and the selected plant species (the "recipient") are chosen such that upon crossing, produce unstable progeny or demonstrate segregation preferential or sorting out. Suitable pairs of plants include the donor, *Tripsacum,* and as the recipient, maize, wheat, barley or oat. In another preferred embodiment, the donor is *Orychophragmus* and the recipient is a crucifer such as canola. Donor/recipient pairs in other preferred embodiments are as follows: *Glycine tomentellal* soybean; *Solanum phreja*/potato; maize/wheat; maize/barley; maize/oat; *Pennisetum*/wheat; *Pennisetum*/barley*; Hordeum bulbosum*/barley; *Hordeum bulbosum*/wheat; *Nicotiana digluta*/*Nicotiana tabacum* and *Oryza minuta*/rice. Crossing the donor with the recipient plant is a viable technique provided that a fertile plant can be regenerated from a tissue culture carrying the PACs. Movement of the PACs from plant cell to yeast cell is accomplished by fusing the plant protoplast with a yeast spheroplast.

In another embodiment, a sexual cross between two plants is used not only to rescue chromosome fragments, but for their subsequent movement between different organisms as well. In this embodiment, transformed plants are produced as described. Instead of treating protoplasts, whole transgenic plants or pollen from a whole transgenic plant is treated with an agent, such as irradiation, to induce chromosome fragmentation. Such treated pollen is then used to pollinate a second plant. It has been shown (Pandey, Nature *256:310-313* (1975)) that crosses between irradiated and normal plants can be done and result in transfer of marker traits from the irradiated organism; the nature of such transgenosis has never been elucidated. The progeny from such crosses is analyzed and chromosome fragments that function as chromosomes are identified in the same way as chromosomes reconstructed through a protoplast fusion.

The exogenous nucleic acid varies widely. For example, it embraces any DNA not present in the native plant genome or in the desired copy number, and that encodes a protein whose expression in plants would be valuable from some standpoint. The DNAs and proteins fall into the broad categories of crop protection, crop improvement, production of specialty compounds including specific chemicals, nutraceuticals and other products associated with food quality such as modified starch, oils and protein compositions, that, in total, require the expression of a coordinate set of genes and thus a specialized transformation system in order to have the plant exhibit the trait of interest. An example is the isoprenoid biosynthetic pathway that is not regulated by plants. All genes involved in mevalonate biosynthesis - - HMG-CoA synthase, HMG-CoA reductase, mevalonate kinase, phosphomevalonate kinase, mevalonate pyrophosphate carboxylase and isopentenyl pyrophosphate isomerase - - are well known. Other examples are the genes involved in amino acid synthesis. The exogenous genes can be useful for modifying the input requirements of a plant such as their response to the environment, their ability to protect themselves from pests, protection from xenobiotic agents, which alter other traits such as overall yield, production of nutritionally balanced protein, better quality starch, high quality or quantity of oil or vitamin levels. The genes may also allow the plants to perform functions they normally do not such as to produce pharmaceutical proteins, antigens and small molecules.

In addition to the gene or genes that are intended to provide a recipient of the plant artificial chromosome with one or more traits of interest, it is preferred that the exogenous nucleic acid is organized in such a fashion to include more characteristics *e.g.*, recombination sites for introducing new genes, components of a yeast artificial chromosome (YAC) such as telomeric sequences and an autonomous replicating sequence and preferably these elements in combination with a centromere, which import yeast-plant shuttle characteristics to the PAC, low frequency restriction enzyme sites for subsequent cloning, and other properties that facilitate subsequent manipulation *in vitro.* The presence of the YAC sequences allows further selection for the presence of artificial chromosomes and for performing genetic manipulations with them. The artificial chromosomes can be transferred to yeast for re-designing, *e.g.* equipping with the sequences of interest, including those for site-specific homologous recombination, and put back into the plant cells. See, U.S. Patents 5,270,201; 5,288,625; 5,721,118; and 5,712,134. Yeast possess an efficient homologous recombination system that facilitates the DNA manipulation within yeast cells (Spencer *et al.,* In Methods: a companion to Methods Enzymol. *5*:161-175, (1993); Hieter *et al.,* in Genome analysis: genetic and physical mapping. Ed. Davies, Tilghman. Cold Spring Harbor: C S H Laboratory Press, *1*:83-120, (1990)). Actually as little as 30 bp of a homologous sequence at each end of a DNA fragment is sufficient to integrate the fragment into a linearized plasmid in yeast *(Hua et al.,* Plasmid, *28*:91-96 (1997)). Development of a yeast artificial chromosome (YAC) makes manipulation with foreign DNA even more convenient. YACs can tolerate more than 2 Mb of DNA inserts (Burke *et al.,* Science, *236*:806-812 (1987)). Plant minichromosomes carrying plant selectable markers and YAC sequences might serve as a shuttle vector that can be transferred from the plant cells into yeast for genetic manipulation and then be returned into the plant cell. By using a homologous recombination system, this shuttle vector can be equipped with any genes of interest, sequences recognized by site-specific recombinases (FLP, R, Cre), or additional selectable markers. Alternatively, large fragments of plant minichromosomes can be deleted or replaced with other sequences (Spencer *et al.,* in *Methods: a companion to Meth. Enzymol. 5*:161-175 (1993)). It also allows preparative isolation of plant minichromosomes for further manipulations such as subcloning and sequencing or re-introduction into the plant cells with the help of microinjection, lipofection or electroporation. However, these transformation methods might have a technical barrier for obtaining sufficient amounts of intact DNA. Direct introduction of YAC DNA by cell fusion was reported for mammalian cells (Pavan *et al.,* Mol. Cell. Biol. 10:4163-4169 (1990); *Pachnis et al.,* Proc. Natl. Acad. Sci USA, *87*:5109-5113, (1990)) as well as for plant protoplasts (Hatsuyama *et al.,* Plant Cell Physiol. 35:93-98 (1994). The latter method allows transferring the DNA of interest from yeast into a plant cell by the means of yeast spheroplast-plant protoplast fusion, thus avoiding DNA fragmentation. The same approach can be used for the minichromosomes transfer from plant cells into the yeast. Thus, the minichromosomes carrying all elements necessary for independent maintenance in plant and yeast cells display all features of plant-yeast shuttle vector.

Site-specific recombinases from bacteriophage and yeasts are being widely used as tools for manipulating DNA both in the test-tube and in living organisms. Preferred recombinases/recombination site combinations for use in the present invention are Cre-Lox, FLP-*FRT*, and R-*RS*, where Cre, FLP and R are recombinases from bacteriophage P1, yeast and *Zygosacharomyces rouxii* respectively, and *Lox, FRT,* and *RS* are the recombination sites. Other suitable systems include the attP and attB sites recognized by integrase of *Streptomyces* bacteriophage phiC31 site-specific recombination system. To be functional in plants, these sites require 7-8 base pairs (bp) of core sequence between 12-13 bp inverted repeats; the asymmetric core site determines the site orientation, and thus the types of recombination product. Regardless of whether recombination sites are placed on or within a single DNA molecule in direct or opposite orientation, or placed on unlinked linear or circular DNA molecules, the corresponding recombinase can catalyze the reciprocal exchange to produce a deletion, inversion, translocation or co-integration event. See, Bollag *et al., Ann. Rev. Genet. 23*:199-225 (1989); Kilby *et al., Trends Genet. 9*:413-421 (1993); and Ow, *Curr. Opinion Biotech. 7*:181-186 (1996). Examples of low frequency restriction sites e.g., for rare-cutting restriction enzymes and nucleases, include intron encoded yeast endonuclease I-*Sce*I (Choulika et al., Mol. Cell Biol. *15*:1968-1973 (1995)), Ho nuclease of *S*. *cerevisiae, Not*1 (an 8 bp cutter) and 6 b.p. cutters with low number of recognition sites in plant genomes e.g., *Sal*1 and *Cla*1*.*

Another embodiment of the present invention is directed to cultures of plant cells containing chromosome fragments containing the exogenous DNA and that exhibit normal chromosomal activities. Another aspect of this embodiment is directed to transgenic plants regenerated or derived from the aforementioned cultures. Plants produced in accordance with the disclosed methods are genetically different from transgenics produced via known methods. The transgenic plants of the present invention contain the transgenes in a single locus and allow for the transgenes to move together as a single locus in a breeding program. Known methods, on the other hand, result in the random integration of the transgene in the plant genome.

The methods of the present invention may be practiced on a wide variety of plants. These include: maize, tomato, turfgrass, asparagus, papaya, sunflower, rye, beans, potato, rice, peanut, barley, malt, wheat, alfalfa, soybean, oat, eggplant, squash, onion, broccoli, sugarcane, sugar beet, beets, apples, oranges, grapefruit, pear, plum, peach, pineapple, grape, rose, carnation, tulip, Douglas fir, cedar, white pine, scotch pine, spruce, peas, cotton, flax, coffee and members of the *Brassica* family such as canola and rape seed.

Although described thus far in the context of plant cells, the method of the present invention also is applicable to animal cells and animal artificial chromosomes. This embodiment entails introducing an exogenous nucleic acid e.g., DNA, into animal cells to produce a transformed animal cell, treating the transformed animal cells e.g., by irradiation or with chemical agents, to produce chromosome fragments; fusing the treated animal cells with non-transformed or undamaged animal cells that are the same or different from the transformed cells (preferably the same or a closely related animal such as from the same family); and identifying cells derived from the fused cells that contain chromosome fragments that exhibit normal animal chromosomal activities and wherein the exogenous nucleic is expressed.

The invention will be further described by reference to the detailed examples. These examples are provided for purposes of illustration only, and are not intended to be limiting unless otherwise specified.

### EXAMPLES

This section contains three examples. Example 1 describes experiments conducted to demonstrate the production of stable microchromosomes following irradiation, fusion and plant regeneration. Example 2 demonstrates the production of stable artificial chromosomes that are able to confer selective advantage to the cells in which they are maintained and which contain yeast artificial chromosomes for conferring shuttle properties to the plant artificial chromosome. Example 3 is a method for visualizing the location of the introduced exogenous DNA in a plant cell and for the visualization of microchromosomes.

### Example 1: Evidence For The Production Of Stable Chromosome Fragments

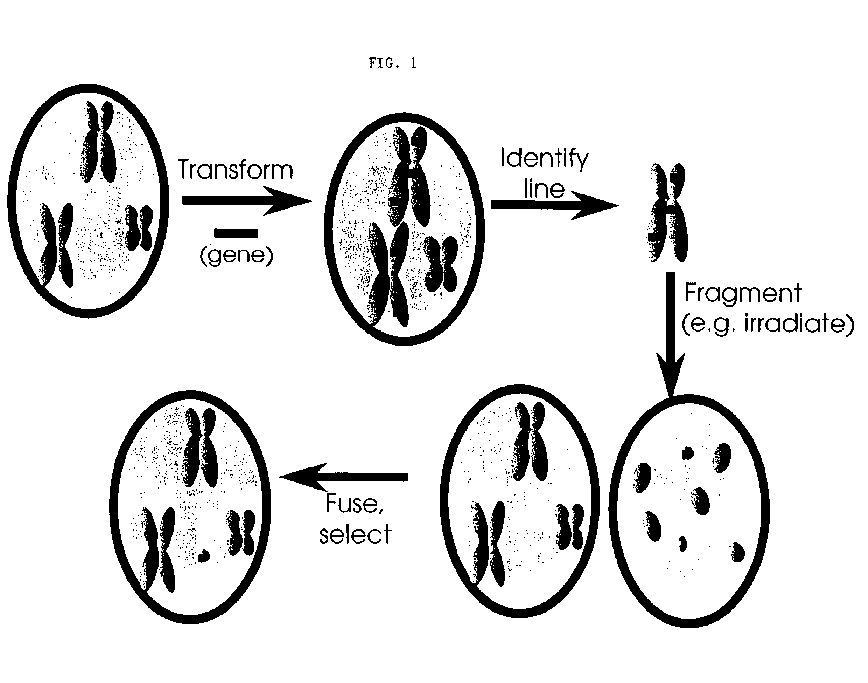

Populations of asymmetric hybrids are produced by fusion with a radiation-inactivated donor. The manipulation of the introduced genetic variation could result in a quicker introgression of a desired trait. Therefore, this technique could be applied in breeding programs. In the asymmetric nuclear hybrids produced between distant and closely related species, the resulting plants were male sterile, even though they appeared to contain only a few chromosomes from the donor partner as shown in Famelaer, *et al.,* Theor. Appl. Genet. 79:513-520 (1990). The scheme presented in Fig. 1 shows the basic strategy applied for the minichromosomes' production in plant cells.

### Protoplast isolation, fusion, selection, and regeneration

Shoot cultures of the NR-deficient mutant, Nia26, of *N. plumbaginijolia* (2n 20, reversion frequency 4.04 10-7 (Dirks *et al.,* Mol. Gen. Genet. *179*:283-288 (1986)) were cultivated as described by Negrutiu, *et al.,* Theor. Appl. Genet. *66*:341-347 (1983). Shoot cultures of *N. sylvestris,* V-42 (2n = 24, chlorophyll-deficient mutant), were cultivated on R'SA medium (Negrutiu, *et al.,* Theor. Appl. Genet. *66*:341-347 (1983)). Mesophyll protoplasts were isolated from both parents according to Negrutiu, Z. Pflanzenphysiol. *100*:373-376 (1981). Donor protoplasts were irradiated in a Gamma cell 200 (Co60 source, dose rate 0.048 J kg-ls-1) with different doses, and fused with recipient cells according to Kao, in: Wetter, LR, Constabel F (eds.) Plant Tissue Culture Methods, NRCC *19876*:49-57, (1982). Culture and selection conditions of protoplasts and fusion products were carried out according to Dirks *et al.,* Mol. Gen. Genet. *179*:283-288 (1986). Cell colonies were regenerated on RPO.25 or RP1 medium (Installe *et al.,* J. Plant Physiol. *119*:443-454 (1985)) with 0.25 mg or 1 mg/l zeatine. Regenerated plants were further cultivated on R'SA medium. Chromosome analysis of regenerated hybrids.

Chromosome analysis was facilitated by morphological differences of recipient and donor metaphase chromosomes:

*N. plumbaginifolia* is characterized by telocentric and *N. sylvestris* by meta- or submetacentric chromosomes. Regenerants can be classified into two groups: a first group of 19 plants from 13 different cell colonies, with 43―56 chromosomes (37 - 42 recipient chromosomes), and a second group (2 plants from 2 independent cell colonies) with 61 - 67 chromosomes and a hexaploid set of recipient chromosomes. The average number of identifiable donor chromosomes in 15 independent regenerants is about 8.7. Donor chromosome fragments resulting from radiation-induced damage were observed in all plants.

The total number and exact type of chromosomes are difficult to establish for several reasons. First, small variations in chromosome numbers may exist within one regenerant. Also radiation damage of donor chromosomes may result in recipient-like chromosomes; and interspecific chromosome exchanges may result in reconstructed and deleted recipient chromosomes as well as in the loss of chromosomes. Finally, chromosome translocations could not be observed.

Fusion products between remote species generally spontaneously eliminate one of the parental genomes, thereby creating asymmetric hybrids that contain, in addition to a complete recipient genome, a few chromosomes derived from the donor. Methods for transferring part of the plant genome have been developed since one often wants to introduce only a small number of traits from the donor into the recipient. The donor-recipient method, also called "gamma" fusion, is the most frequently used technique for creating asymmetric somatic hybrids. Although irradiation directs the process of chromosome elimination, it is not the sole control of the process. Highly asymmetric hybrids in which only one or few donor chromosomes are contained, have only rarely been described. This observation is irrespective of the radiation dose use.

### Minichromosomes production in asymmetric "gamma" hybrids between Nicotiana and Atropa

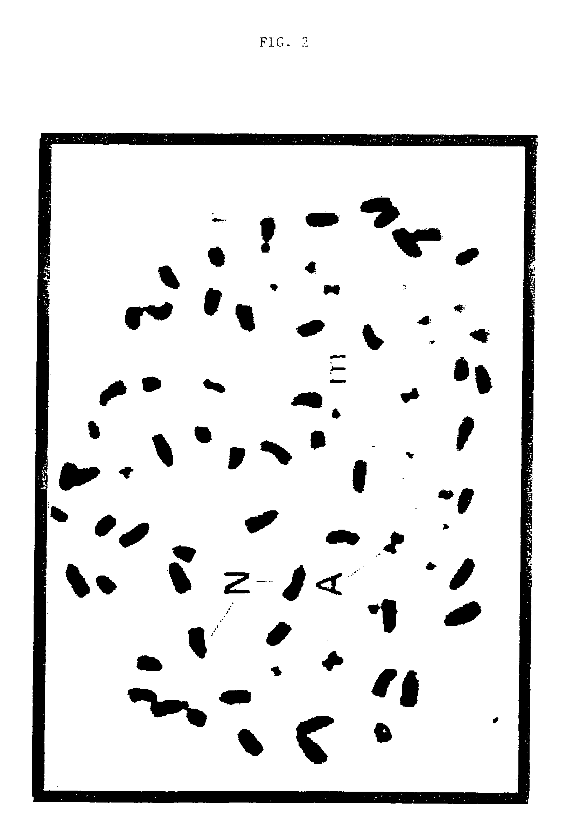

Nitrate reductase deficient mutant of *Nicotiana plumbaginifolia* (cnx20) and *Atropa belladonna* plants were used for the experiments. Protoplasts were isolated as described by *Negrutiu et al.,* Theor. Appl. Genet. 72:279-286 (1986). Treatment *of Atropa belladonna* protoplasts with different doses of gamma rays was performed as described by *Gleba et al.,* Theor. Appl. Genet. 76:760-766 (1988). The fusions of protoplasts were carried out as described by Menczel *et al.,* Genetics, *100*:487-495 (1982). Protoplasts were cultured in K3 medium for 2 weeks, diluted in MDn medium (Negrutiu *et al.,* Theor. Appl. Genet. 66:341-347 (1983); Negrutiu *et al.,* Theor. Appl. Genet. *72*:279-286 (1986)). After 1 month visible calli were transferred to solid medium and regenerated as described elsewhere (Installe *et al.,* J. Plant Physiol. *119*:443-454 (1985)). Metaphase plates from root tips for chromosome analysis were prepared as described by Gleba *et al.,* Theor. Appl. Genet. *76*:760-766 (1988). The chromosomes analysis was greatly facilitated as the *Atropa* metaphase chromosomes are approximately twice shorter and significantly thinner that *Nicotiana plumbaginifolia* chromosomes (Gleba *et al.,* Theor. Appl. Genet. *76*:760-766 (1988)). Minichromosomes, which are significantly smaller than *Atropa* intact chromosomes, were detected in all samples analyzed. The size of detectable minichromosomes was variable starting from almost half the size of parental chromosome to hardly visible minichromosomes (Fig. 2). It is very possible that such "gamma" hybrids contain so small minichromosomes that they are simply invisible on the metaphase plate.

The constructs described in Example 1 suit many different purposes including plant minichromosome rescue in yeast cells and cloning any genes of interest. The genes of interest which could be cloned into the YAC would include, but not be limited to, genes for herbicide resistance, quality trait improvement such as starch modification, oil quality, protein quality, drought resistance, cold tolerance, pest resistance, and other input and output agricultural traits.

Any number of methods can be used to identify the integration sites of the YAC. These methods include RAPD to mapping, fluorescent *in situ* hybridization (FISH) and Southern analysis. By identifying constructs that have inserted near the centromere, there is a greater chance of recovering fragmented chromosomes containing the centromere region and the construct of interest. There is evidence that this minimal "chromosome" can be repaired in the cell to include telomeres. This biologically assembled artificial chromosome could be rescued by transformation into yeast and from them being used to re-transform other plants of the same species.

### Example 2: Identification Of Kanamycin Resistant Plants Following Irradiation And Asymmetric Fusion

### Isolation, fusion and culture of protoplasts

Mesophyll protoplasts were isolated from 4- to 6-week-old plants of *N. plumbaginifolia* (P2) and kanamycin-resistant *Petunia hybrida* (transformant VR2828 x V23) as described by Negrutiu *et al.,* Theor. Appl. Genet. 72:279-286 (1986). Before fusion kanamycin-resistant Petunia protoplasts were irradiated with gamma rays (100 krad) from a cobalt60 source. Fusions were carried out as described by Menczel et al., Genetics *100:487-*495 (1982). The protoplasts were further cultured in K3 medium and subsequently diluted in selection medium (MDn) supplemented with 25 mg/1 kanamycin monosulphate. After one to two months, visible calli were transferred to solid selection medium and subsequently regenerated as described by Installe *et al.,* J. Plant Physiol. *119*:443-454 (1985). Control experiments were carried out under the same conditions. Cytological analysis

For chromosome analysis, metaphase plates were prepared using the protoplast method as described by Mouras *et al.,* Caryolgia *31*:117-127 (1978). Alternatively, metaphase spreads were also obtained from the root tips of regenerated plants as described by Pijnacker *et al.,* Can. J. Genet. Cytol. *26*:415-419 (1984).

### Hybrid isolation

One to two weeks after fusion of wild-type *N. plumbaginifolia* (P2) protoplasts with irradiated (100 krad) kanamycin-resistant *Petunia hybrida* (transformant VR2828 x V23) protoplasts, kanamycin monosulphate, at a concentration of 25 mg/l, was added to the culture medium. After a further culture of one to two months, green resistant calli were obtained at a frequency of about 10-4. This transformation frequency is in the range of "gamma"-fusion experiments. In total, 86 stable kanamycin-resistant calli were recovered and 24 (28%) could easily be regenerated into plants that resembled the recipient partner *N*. *plumbaginifolia.* Moreover, organogenic hybrid calli regenerated numerous shoots that were analyzed at the cytological, molecular and genetic levels. As expected, control experiments did not result at all in the production of resistant colonies on the selection medium.

A total of 14 lines have been analyzed by karyotypic analysis. In most of the lines only *Nicotiana* chromosomes were observed at the diploid or tetraploid level. However, in one diploid line and four lines that were nearly tetraploid, a few (2-3) chromosome fragments could be seen.

A number of asymmetric nuclear hybrid clones have been produced by the treatment of plant cells with lethal doses of irradiation and subsequent fusion experiments. From these results it seems that irradiation can be used to partially direct the process of chromosome elimination, and that the elimination is not only due to the mutagenic effect of the irradiation. This process results in completely fertile hybrid plants. The ability to obtain hybrid plants that were kanamycin resistant was dependent on the presence of the kanamycin gene in the irradiated donor. All of the regenerated plants resembled the recipient partner. In some lines the presence of a few chromosome fragments was demonstrated, which is probably created by the irradiation of the protoplasts.

### Vectors for minichromosome tagging

In order to tag and rescue plant minichromosomes produced as described above, two different vector systems were used.

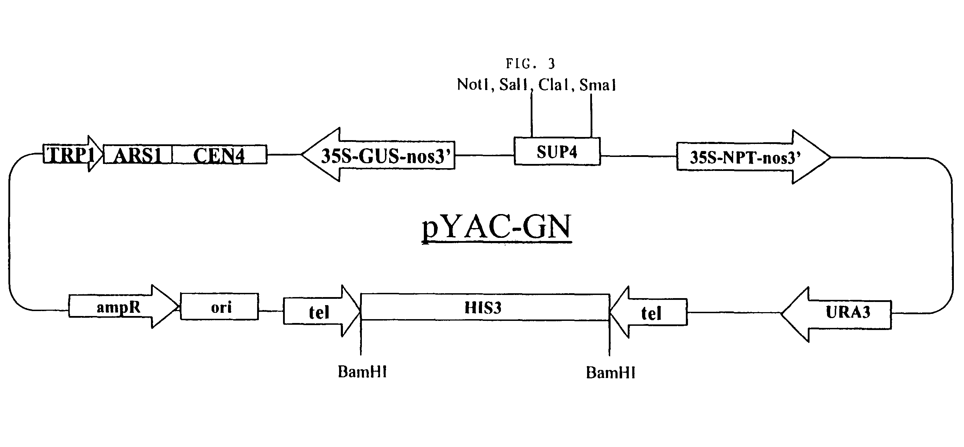

The first vector pYAC-GN is shown in Fig. 3. It was created from pYAC-4 by insertion of two genes: p35S-APH(3')III- NOS3' and p35S-GUS-NOS3' into *Sall* and *Clal* sites respectively. Transformed plants can be selected for kanamycin resistance as well as for GUS activity. A Polylinker containing rare-cutting for plant DNA sites was inserted into *EcoR1* site of SUP4 gene. This polylinker can be used for further modification of pYAC-GN by inserting for example recombination sites recognized by site-specific recombinases, thus allowing integration of any gene(s) of interest into plant minichromosomes containing YAC-GN. The rest of the sequences in the construct are of pYAC 4 origin and can be used to resque plant minichromosomes in yeast cells.

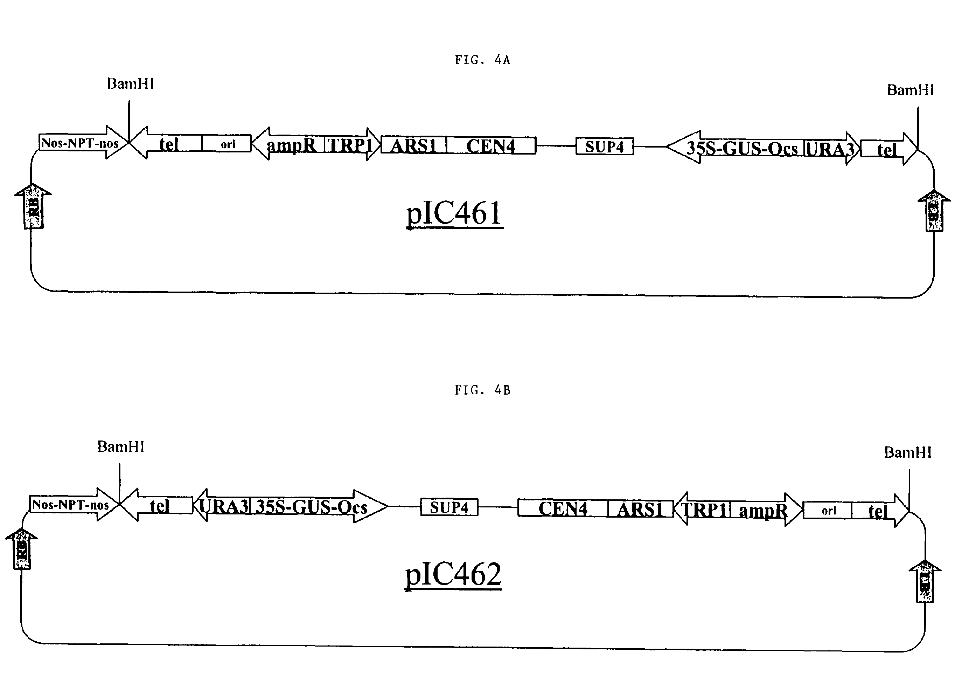

Two other vectors, pIC461 and pIC462 (Fig. 4) were made as described below. Expression cassette p35S:GUS:OCS3' was cloned into *ClaI* site of pYAC-4. Resulting plasmid was inserted into pBIN19 as *Bam*HI fragment, producing pIC461 or pIC462 depending on orientation of insert. This vector allows *Agrobacterium-*mediated transformation of plant cells due to the presence of pNOS-NPTII-NOS3' gene within T-DNA borders.

### Transformation of Nicotiana species with YAC-derived constructs.

The vector pYAC-GN was used for direct transformation of *Nicotiana tabacum* protoplasts using a solution of PEG with Ca²⁺ and pH 9.8. Seeds of *Nicotiana tabacum cv* Wisconsin were germinated on media MS after sterilization with solution "diacid" for 5 minutes and washing 5 times with sterile water. Three- to four-week old leaves were used for protoplast isolation. The protoplasts were transformed as described by Koop *et al.,* Planta, *199*:193-201 (1996). Leaves were cut on pieces and put abaxial side down on the surface of filter-sterilized enzyme solution containing Cellulose Onozuka 0.5%, Macerozime 0.5%, Dricelase 0.25%, Cellulysine 0.25%, 0.5 M mannitol and CaCl₂ * 2 H₂O 110 mg/l. The pH was adjusted to 5.7. After a 16h incubation at 27 °C in the dark, leaf pieces were teased to release any protoplasts that had not been liberated by enzyme action alone. The protoplasts were sedimented by centrifugation (100xg, for 5 min) and washed in 0.5M sucrose, 15mM CaCl₂. W5 solution was loaded onto the top to prevent damage of protoplasts from direct contact with the air. After centrifugation at 100xg for 5 min protoplasts were transferred to a new centrifuge tube and resuspended in 10 ml of TB buffer containing 0.4 M mannitol, 15 mM CaCl₂ pH 5.7. The density of protoplasts was adjusted to 5x10⁶ protoplasts/ml. One hundred microliters of this suspension was transferred to 6-cm Petri dish and the protoplasts were left for a few minutes to settle. The DNA solution (25µl of 50µg pYAC-GN dissolved in 18 µl TE pH5.6 plus 7µl of culture media) was carefully added to the suspension, resuspended by gentle shaking of Petri dish and finally mixed with 125µl of PEG solution (40% (w/v) of PEG 4000, 70mM Ca(NO₃)_{2,} 1,2754g mannitol per 26 ml). After 7-8 min of incubation the culture media was added to a volume of 125µl, and after two minutes 2.6 ml of culture media was gradually added. After two weeks of incubation kanamycin was added to select transformed colonies. The *Agrobacterium*-mediated plant transformation was used with two other vectors -- pIC461 and pIC462. Leaf-discs transformation of *Nicotiana sylvestris* and *Nicotiana tabacum* species was performed using standard protocol. Leaf disks were co-cultivated with *Agrobacterium tumefaciens* EHA105 harboring pIC461 or pIC462 in MS media complemented with 1mg/l BAP and 0.5 mg/1 NAA. After 24 hours leaf-discs were transferred onto solid MS media with 1mg/l BAP, 0.1 mg/L NAA, 200mg/L carbeniciline and 200mg/L cefotaxime. After 7 days explants were transferred onto the same media but complemented with 25mg/L kanamycin. After 2-3 weeks concentration of kanamycin was increased to 50mg/l.

### Generation of minichromosomes by irradiation of protoplasts.

Protoplasts of primary transformants carrying pYAC-GN or T-DNA of either pIC461 or pIC462 were used for gamma-irradiation. Then gamma-irradiated protoplasts of *Nicotiana tabacum* were fused with protoplasts from the other. It was reported in Parokonny *et al.,* Plant *J., 2*:863-874 (1992), that a number of stable minichromosomes originated from the irradiated parent was present in several regenerants. The purpose of this experiment, therefore, was to get the asymmetric somatic hybrid having a minichromosome with loxP site. Two irradiation doses were used - - 250Gy and 500Gy. The protoplast fusion technique performed was based on that of Negrutiu *et al.,* Theor. Appl. Genet. *66*:341-347 (1983). Leaves were cut on pieces and put abaxial side down on the surface of filter-sterilized enzyme solution containing Cellulose Onozuka 0.5%, Macerozime 0.5%, Dricelase 0.25%, Cellulysine 0.25%, 0.5 M mannitol, CaCl₂ * 2 H₂O 110 mg/l, 6mg/l BAP and 2mg/l NAA. The pH was adjusted to 5.7. After 16h of incubation at 27 °C in the dark the protoplasts were pelleted by centrifugation (100g, for 5 min.) and washed in the solution of 0.5M sucrose with 15mM CaCl₂, W5 was loaded onto the top of suspension in order to prevent protoplasts from contact with air. Protoplasts were resuspended in 10 ml of W5 and irradiated. Irradiated protoplasts were mixed with non- irradiated protoplasts of *Nicotiana plumbaginifolia.* Approximately 0.5 ml of that mix were transferred to the 6-cm Petri dish and allowed to settle for 20 minutes. Equal volume of PEG solution [40% PEG 4000, 70mM Ca (NO₃)₂, 1,2754g mannitol per 26 ml (0.27M)] was carefully added Then 200µl of W5 were added, incubated for 15-30 min. and mixed with another 2 ml of W5. After 20 minutes of incubation W5 was changed once and finally replaced with 3 ml of culture media. After two to three weeks of incubation, kanamycin sulfate was added to select hybrid colonies. The selected hybrids carried complete *Nicotiana plumbaginifolia* genome and minichromosomes with YAC sequences and kanamycine resistant gene.

The same procedure was applied to produce asymmetric somatic hybrids between irradiated protoplasts of *Nicotiana tabacum* and *Nicotiana sylvestris* transformed with pIC461, pIC462 or pYAC-GN and protoplasts *of Nicotiana plumbaginifolia.*

### Example 3: Genomic in situ hybridization (GISH).

In the process of producing asymmetric somatic hybrids is necessary to unequivocally identify the alien DNA and recipient genome. Methods used in the past include the analysis chromosomal genes, marker genes, and species-specific repeat sequences. The use of cytogenetic markers to identify chromosomes in chromosomal segments was limited to chromosomes that differed significantly in size or morphology.

The use of genomic *in situ* hybridization in which total genomic DNA used as a probe can be used to determine the parental origin of chromosomal material in asymmetric hybrids as described in Parokonny *et al.,* Plant Journal *2*:863-874 (1992).

### Experimental procedures:

### Plant material

Shoot cultures of nitrate reductase-deficient mutants of *N. plumbaginifolia* (cnx 20 and Nia 26, both with 2n = 20), and a chlorophyll-deficient mutant of *N. sylvestris* CV-42, 2n = 24) were cultivated as described by Negrutiu *et al.* Theor. Appl. Genet. *66*:341-347 (1983). An autotetraploid cytotype of *N. plumbaginifolia* (2n = 4x = 40), used as a control for *in situ* hybridization, was generated by somatic doubling from wild-type material. Wild-type *A. belladonna* introduced into *in vitro* culture was used as a control for dot blot hybridizations. All material was originally raised at the Institute of Cell Biology and Genetic Engineering, Kiev, and maintained in *in vitro* culture at the Jodrell Laboratory, Royal Botanic Gardens, Kiev.

Asymmetric somatic hybrids were obtained by cell fusion of leaf mesophyll protoplasts from the non-irradiated parent (recipient) with gamma-irradiated mesophyll protoplasts from the other (donor). Irradiation doses administered prior to fusion ranged between 10 and 1000 Gy. Regenerants originated from individual nuclear hybrid colonies of the different fusion combinations. Regenerant Oct-3 was a product of symmetric cell fusion between mesophyll protoplasts of *N. plumbaginifolia* cnx 20 and a lysine-overproducing mutant of *N. sylvestris* (ALC; Negrutiu *et al.,* Theor. Appl. Genet. *68*:11-20 (1981)), without prior irradiation of one of the parents (Famelaer *et al.,* Plant Sci. *61*:105-117 (1989)). Regenerants were cultivated as described by Negrutiu *et al.,* Theor Appl. Genet. *66*:341-347 (1983) and Korostash *et al.* Biopolymers and the Cell *7*:55-62 (1991).

### DNA isolation

Approximately 0.5-1 mg of genomic DNA were extracted from three to four young leaves of *N. plumbaginifolia* cnx 20 and *N. sylvestris* V-42, using the minipreparation technique of Dvorak, *et al.,* Theor. Appl. Genet *63*:349-360 (1982).

### Dot blot hybridization

Samples containing between 0.02 g and 0.4 g of genomic DNA were denatured in 0.4 M NaOH, 10 mM EDTA, boiled for 10 min. and neutralized in 3 M ammonium acetate (pH 7.0). These were loaded into different wells of a BioRad dot-blotter in a final volume of 100 1 each. Identical dilution series were prepared for DNA from *N*. *sylvestris, N. plumbaginifolia* and *A. belladonna.* Blots were hybridized with 0.5 g ml-1 biotinylated total genomic DNA from *N. sylvestris* v-42, using the method described by Parokonny *et al.,* Plant J. 2:695-704 (1992). Labeled DNA was detected using the BRL DNA detection kit (Life Technologies).

### DNA probes

### Total genomic DNA

For use as a probe for GISH, 3 g of total genomic DNA were sheared by vortexing for 10-30 sec. before labeling as described below.

(TTTAGGG)n. A high molecular weight probe concatenated by the polymerase chain reaction (PCR) from a synthetic oligomer homologous to the consensus sequence of the *A. thaliana* telomeric repeat (5'-TTTAGGG-3'; Richards, *et al.,* Cell *53*:127-136 (1988)), was kindly supplied by A.V. Cox (Jodrell Laboratory, Royal Botanic Gardens, Kiev).

pTa71. A probe containing the 5.8S, 18S and 25S ribosomal genes, and part of the intergenic spacer from wheat (pTa71; Gerlach, *et al.,* Nucl. Acids Res. *7*:1869-1885 (1979)), recloned into pUC18, was kindly supplied by Dr Kevin Jones, Department of Botany, University of Reading.

### DNA probe labeling

Probe DNA was labeled with biotin-14-dATP (Life Technologies, Paisley, UK) by nick translation, as recommended by the manufacturer. Unincorporated nucleotides were removed by spin dialyzing through a 200 1 Sepharose CL-6B (Pharmacia) column *(Maniatis et al., Molecular Cloning: A Laboratory Manual.* Cold Spring Harbor. Cold Spring Harbor Laboratory Press, 1982). If the probe was to be used for *in situ* hybridization, a 30x excess of sheared denatured salmon testis DNA was added at this stage. The mixture was then precipitated once in ethanol and reconstituted in 20 1 of TE (10 mM Tris-HCI, pH 8.0,1 mM EDTA). Spotting an aliquot of labeled DNA onto nitrocellulose and detecting labeled DNA using the BRL DNA detection kit (Life Technologies) tested biotin incorporation.

### Chromosome preparations

Root tips were pretreated in 0.035% colchicine (Sigma, Poole, UK) for 1.5 hours at room temperature, fixed in 3:1 ethanol:acetic acid for up to 2 weeks at 4°C, and stored in 70% ethanol at -20C. Root tips were squashed in 45% acetic acid on slides treated with Vectabond (Vector Laboratories, Peterborough, UK) to aid cell adhesion. Slides were stored desiccated at -20C for one to two weeks before processing. They were then immersed in 3:1 ethanol:acetic acid for 30 min. and absolute ethanol for 2 x 10 min., air-dried and treated with 50 g ml-1 of DNase-free RNase in 2 x SSC for 2 hr. at 37°C. After dehydrating through an alcohol series, they were dried in a vacuum desiccator overnight at 4°C.

### In situ hybridization

The method used for *in situ* hybridization was as described by Parokonny *et al.,* Plant J. 2:695-704 (1992). The hybridization mixture consisted of 10% dextran sulphate, 50% formamide, 450 g ml-1 sonicated, sheared salmon sperm DNA and biotinylated probe to final concentrations of 15 g ml-1, 5 g m-1 and 200 ng ml-1, respectively, for GISH, (TTTAGGG)n and pTa71. Post-hybridization washes were in 2 x SSC at 42C, 50% formamide, 50% 2 x SSC at 42°C and 2 x SS°C at room temperature. Biotinylated DNA was detected with fluoresceinated avidin using one amplification with biotinylated anti-avidin D as described by Schwarzacher *et al.,* Ann. Bot. *64*:315-324 (1989). Unhybridized DNA was visualized by staining with 0.5 g ml-1 propidium iodide. Chromatin from both parents was detected by counterstaining with 2 g ml-1 diaminidophenylindole (DAPI). Fluorescence was viewed with an Axiophot microscope (Carl Zeiss, Oberkochen, Germany), using Zeiss filter block 9 (excitation 450-490 nm) for simultaneous detection of fluoresceinated avidin and propidium iodide, and Zeiss filter block 1 (excitation 365 nm) for DAPI.

GISH was used to localize, and from each parental species in metaphase spreads of asymmetric somatic hybrids. Each of 31 plants originating from a different nuclear hybrid was treated by GISH. The irradiated donor for 17 of these was *N. sylvestris* and the remaining 14 were *N. plumbaginifolia.* The chromosomes from *N. sylvestris* fluoresced yellow, and those from *N. plumbaginifolia* fluoresced red. By the color of their fluorescence the species origin in rearranged chromosomes could be determined unequivocally. By counterstaining with DAPI, minichromosomes were identified and the donor chromosome could be identified by the color of the fluorescence. *In situ* hybridization localized the telomeric repeat at the termini of all chromosomes. Signals for the telomeric region could also be seen on chromosomes in which large segments had been deleted following irradiation.

### INDUSTRIAL APPLICABILITY

The present invention is applicable to agricultural biotechnology and in particular, to the manipulation of the plant genome to produce plant artificial chromosomes and the introduction of non-native nucleic acid into plants using the artificial chromosomes.

All publications mentioned in this specification are indicative of the level of skill of persons skilled in the art to which this invention pertains.

Various modifications of the invention described herein will become apparent to those skilled in the art. Such modifications are intended to fall within the scope of the appending claims.

## Claims

1. A method of making a plant artificial chromosome, comprising:
(a) preparing recombinant protoplasts of a first plant species containing an exogenous nucleic acid comprising a gene of interest;
(b) producing chromosome fragments of chromosomes contained in the recombinant protoplasts;
(c) fusing the recombinant protoplasts of (b) with protoplasts of a second plant species to produce fused protoplasts, wherein the first and second plant species may be the same or different; and
(d) identifying fused protoplasts of (c) or cells derived from the fused protoplasts of (c) that contain chromosome fragments comprising the gene of interest and exhibiting normal plant chromosomal activities.

2. The method of claim 1 wherein (b) comprises irradiating the protoplasts.

3. The method of claim 1 wherein (b) comprises contacting the protoplasts with a chemical agent.

4. The method of claim 3 wherein the chemical agent is calicheamicin, esperamicin, dynemicin or neocarzinostatin.

5. A method according to any one of claims 1 to 4 wherein said identifying of (d) comprises pulsed field gel electrophoresis.

6. A method according to any one of claims 1 to 5 wherein said second plant species is the same as said first plant species.

7. A method according to any one of claims 1 to 5 wherein said second plant species is a member of the same family as said first plant species.

8. A method according to any one of claims 1 to 7 wherein said first plant species is a monocot.

9. A method according to any one of claims 1 to 7wherein said first plant species is a dicot.

10. A method according to any one of claims 1 to 9 further comprising (e) regenerating a whole plant from the recombinant protoplasts of (a), prior to (b).

11. A method according to any one of claims 1 to 9 further comprising (f) regenerating a whole plant from the fused protoplasts or plant cells identified in claim 1 (d).

12. A method according to any one of claims 1 to 11 wherein the exogenous nucleic acid comprises at least one recombination site.

13. A method according to any one of claims 1 to 12 wherein the exogenous nucleic acid comprises at least one restriction site.

14. A method according to any one of claims 1 to 13 wherein the exogenous nucleic acid comprises at least one coding region.

15. A method according to any one of claims 1 to 14 wherein the exogenous nucleic acid comprises at least one sequence comprising a yeast chromosomal element.

16. A method according to any one of claims 1 to 15 wherein the exogenous nucleic acid comprises a yeast artificial chromosome.

17. A method according to any one of claims 1 to 16 wherein the exogenous nucleic acid comprises a sequence encoding a selectable marker.

18. A method of preparing a transgenic plant comprising:
(a) preparing recombinant protoplasts of a first plant species containing an exogenous nucleic acid comprising a gene of interest;
(b) producing chromosome fragments of chromosomes contained in the recombinant protoplasts;
(c) fusing the recombinant protoplasts of (b) with protoplasts of a second plant species to produce fused protoplasts, wherein the first and second plant species may be the same or different;
(d) identifying fused protoplasts of (c) or cells derived from the fused protoplasts of (c) that contain chromosome fragments that exhibit normal plant chromosomal activities; and
(e) regenerating a whole plant from the protoplasts or cells identified in (d) that contain said chromosome fragments comprising the gene of interest and exhibiting normal plant chromosomalactivities.

19. A method of making a plant artificial chromosome, comprising:
(a) producing transformed plants of a first plant species containing an exogenous nucleic acid comprising a gene of interest;
(b) producing chromosome fragments of chromosomes of said first plant species;
(c) crossing said first plant species containing the chromosome fragments with a second plant species to produce hybrid plant species wherein said first and second plant species may be the same or different; and
(d) identifying hybrid plant species of (c) or cells or protoplasts thereof containing at least one chromosome fragment comprising the gene of interest and exhibiting normal plant chromosomal activities.

20. Isolated plant cells or protoplasts containing at least one plant chromosome fragment exhibiting normal chromosomal activities produced by the method of claim 1, wherein the chromosome fragment comprises the gene of interest.

21. A culture of protoplasts or plant cells identified in claim 1 (d).

22. A whole plant produced by the method of claim 11.

23. The whole plant of claim 22 that is a monocot plant.

24. The whole plant of claim 22 that is a dicot plant.

25. Seed derived from the whole plant of claim 22, wherein the seed comprises the gene of interest.

26. The whole plant regenerated by the method of claim 18.

27. A plant cell culture derived from the whole plant of claim 25.

28. Seed derived from the whole plant of claim 26.

29. A hybrid plant species or cells or protoplasts thereof containing at least one chromosome fragment that exhibits normal plant chromosomal functions, produced by the method of claim 19, wherein the chromosome fragment comprises the gene of interest.

30. The method of claim 15, wherein the yeast chromosomal element comprises a first centromeric sequence functional in a yeast cell.

31. The method of claim 30, wherein the chromosome fragments further comprise a second centromeric sequence functional in a plant cell.

32. The method of claim 17, wherein the exogenous nucleic acid further comprises at least one yeast chromosomal element.

33. The method of claim 32, wherein the yeast chromosomal element comprises a first centromeric sequence functional in a yeast cell.

34. The method of claim 33, wherein the chromosome fragment comprises a second centromeric sequence functional in a plant cell.

35. A recombinant nucleic acid comprising a first centromeric sequence functional in a yeast cell, and a second centromeric sequence functional in a plant cell, isolated from the fused protoplasts of (d) in claim 31, or of (d) in claim 34, wherein said recombinant nucleic acid exhibits normal plant chromosomal activities.

## Patentansprüche

1. Verfahren zur Herstellung eines künstlichen Pflanzenchromosoms, welches umfasst
(a) Herstellen von rekombinanten Protoplasten von einer ersten Pflanzenspezies, welche eine exogene Nukleinsäure, welche ein Gen von Interesse umfasst, enthalten;
(b) Erzeugen von Chromosomenfragmenten von Chromosomen, die in den rekombinanten Protoplasten enthalten sind;
(c) Fusionieren der rekombinanten Protoplasten von (b) mit Protoplasten einer zweiten Pflanzenspezies, um fusionierte Protoplasten zu erzeugen, wobei die erste und die zweite Pflanzenspezies gleich oder unterschiedlich sein können; und
(d) Identifizieren von fusionierten Protoplasten von (c) oder von Zellen, die von den fusionierten Protoplasten von (c) abgeleitet sind, die Chromosomenfragmente enthalten, welche das Gen von Interesse umfassen und normale pflanzenchromosomale Aktivitäten zeigen.

2. Verfahren nach Anspruch 1, wobei (b) ein Bestrahlen der Protoplasten umfasst.

3. Verfahren nach Anspruch 1, wobei (b) ein Inkontaktbringen der Protoplasten mit einem chemischen Mittel umfasst.

4. Verfahren nach Anspruch 3, wobei das chemische Mittel Calichcamicin, Esperamicin, Dynemicin oder Neocarzinostatin ist.

5. Verfahren nach einem der Ansprüche 1 bis 4, wobei das Identifizieren von (d) eine Puls-Feld-Gelelektrophorese umfasst.

6. Verfahren nach einem der Ansprüche 1 bis 5, wobei die zweite Pflanzenspezies die gleiche wie die erste Pflanzenspezies ist.

7. Verfahren nach einem der Ansprüche 1 bis 5, wobei die zweite Pflanzenspezies ein Mitglied der gleichen Familie wie die erste Pflanzenspezies ist.

8. Verfahren nach einem der Ansprüche 1 bis 7, wobei die erste Pflanzenspezies eine monokotyledone Pflanzenspezies ist

9. Verfahren nach einem der Ansprüche 1 bis 7, wobei die erste Pflanzenspezies eine dikotyledone Pflanzenspezies ist.

10. Verfahren nach einem der Ansprüche 1 bis 9, welches des Weiteren (e) ein Regenerieren einer vollständigen Pflanze aus den rekombinanten Protoplasten von (a) vor (b) umfasst.

11. Verfahren nach einem der Ansprüche 1 bis 9, welches des Weiteren (f) ein Regenerieren einer vollständigen Pflanze aus den fusionierten Protoplasten oder Pflanzenzellen, die in Anspruch 1 (d) identifiziert werden, umfasst.

12. Verfahren nach einem der Ansprüche 1 bis 11, wobei die exogene Nukleinsäure wenigstens eine Rekombinationsstelle umfasst.

13. Verfahren nach einem der Ansprüche 1 bis 12, wobei die exogene Nukleinsäure wenigstens eine Restriktionsstelle umfasst.

14. Verfahren nach einem der Ansprüche 1 bis 13, wobei die exogene Nukleinsäure wenigstens eine kodierende Region umfasst.

15. Verfahren nach einem der Ansprüche 1 bis 14, wobei die exogene Nukleinsäure wenigstens eine Sequenz, welche ein chromosomales Element aus Hefe umfasst, umfasst.

16. Verfahren nach einem der Ansprüche 1 bis 15, wobei die exogene Nukleinsäure ein künstliches Hefe-Chromosom umfasst.

17. Verfahren nach einem der Ansprüche 1 bis 16, wobei die exogene Nukleinsäure eine Sequenz, welche einen selektierbaren Marker kodiert, umfasst.

18. Verfahren zum Herstellen einer transgenen Pflanze, welches umfasst:
(a) Herstellen von rekombinanten Protoplasten von einer ersten Pflanzenspezies, welche eine exogene Nukleinsäure, welche ein Gen von Interesse umfasst, enthalten;
(b) Erzeugen von Chromosomenfragmenten von Chromosomen, die in den rekombinanten Protoplasten enthalten sind;
(c) Fusionieren der rekombinanten Protoplasten von (b) mit Protoplasten einer zweiten Pflanzenspezies, um fusionierte Protoplasten zu erzeugen, wobei die erste und die zweite Pflanzenspezies gleich oder unterschiedlich sein können;
(d) Identifizieren von fusionierten Protoplasten von (c) oder von Zellen, die von den fusionierten Protoplasten von (c) abgeleitet sind, die Chromosomenfragmente enthalten, welche normale pflanzenchromosomale Aktivitäten zeigen; und
(e) Regenerieren einer vollständigen Pflanze aus den Protoplasten oder Zellen, die in (d) identifiziert werden, die die Chromsomenfragmente, welche das Gen von Interesse umfassen und normale pflanzenchromosomale Aktivitäten zeigen, enthalten.

19. Verfahren zum Herstellen eines künstlichen Pflanzenchromosoms, welches umfasst;
(a) Erzeugen von transformierten Pflanzen von einer ersten Pflanzenspezies, welche eine exogene Nukleinsäure, welche ein Gen von Interesse umfasst, enthalten;
(b) Erzeugen von Chromosomenfragmenten von Chromosomen von der ersten Pflanzenspezies;
(c) Kreuzen der ersten Pflanzenspezies, welche die Chromosomenfragmente enthält, mit einer zweiten Pflanzenspezies, um hybride Pflanzenspezies zu erzeugen, wobei die erste und die zweite Pflanzenspezies gleich oder unterschiedlich sein können; und
(d) identifizieren von hybriden Pflanzenspezies von (c) oder von Zellen oder Protoplasten davon, welche wenigstens ein Chromosomenfragment, welches das Gen von Interesse umfasst und normale pflanzenchromosomale Aktivitäten zeigt, enthalten.

20. Isolierte Pflanzenzellen oder Protoplasten, enthaltend wenigstens ein Pflanzenchromosomenfragment, welches normale chromosomale Aktivitäten zeigt, hergestellt durch das Ver fahren von Anspruch 1, wobei das Chromosomenfragment das Gen von Interesse umfasst.

21. Kultur von Protoplasten oder Pflanzenzellen, die in Anspruch 1 (d) identifiziert werden.

22. Vollständige Pflanze, die durch das Verfahren von Anspruch 1 erzeugt wird.

23. Vollständige Pflanze nach Anspruch 22, die eine monokotyledone Pflanze ist.

24. Vollständige Pflanze nach Anspruch 22, die eine dikotyledone Pflanze ist.

25. Saatgut, welches von der vollständigen Pflanze von Anspruch 22 abgeleitet ist, wobei das Saatgut das Gen von Interesse umfasst.

26. Vollständige Pflanze, die durch das Verfahren von Anspruch 18 regeneriert worden ist.

27. Pflanzenzellkultur, welche von der vollständigen Pflanze von Anspruch 25 abgeleitet wird.

28. Saatgut, welches von der vollständigen Pflanze von Anspruch 26 abgeleitet wird.

29. Hybride Pflanzenspezies oder Zellen oder Protoplasten davon, enthaltend wenigstens ein Chromosomenfragment, welches normale pflanzenchromosomale Funktionen zeigt, erzeugt durch das Verfahren von Anspruch 19, wobei das Chromosomenfragment das Gen von Interesse umfasst.

30. Verfahren nach Anspruch 15, wobei das chromosomale Element aus Hefe eine erste Zentromersequenz, welche in einer Hefezelle funktionsfähig ist, umfasst.

31. Verfahren nach Anspruch 30, wobei die Chromosomenfragmente des Weiteren eine zweite Zentromersequenz, welche in einer Pflanzenzelle funktionsfähig ist, umfassen.

32. Verfahren nach Anspruch 17, wobei die exogene Nukleinsäure des Weiteren wenigstens ein chromosomales Element aus Hefe umfasst.

33. Verfahren nach Anspruch 32, wobei das chromosomale Element aus Hefe eine erste Zentromersequenz, welche in einer Hefezelle funktionsfähig ist, umfasst.

34. Verfahren nach Anspruch 33, wobei das Chromosomenfragment eine zweite Zentromersequenz, welche in einer Pflanzenzelle funktionsfähig ist, umfasst.

35. Rekombinante Nukleinsäure, umfassend eine erste Zentromersequenz, welche in einer Hefezelle funktionsfähig ist, und eine zweite Zentromersequenz, welche in einer Pflanzenzelle funktionsfähig ist, isoliert aus den fusionierten Protoplasten von (d) in Anspruch 31 oder von (d) in Anspruch 34, wobei die rekombinante Nukleinsäure normale pflanzenchromosomale Aktivitäten zeigt.

## Revendications

1. Procédé de fabrication d'un chromosome artificiel de plante, comprenant
(a) la préparation de protoplastes recombinés d'une première espèce végétale contenant un acide nucléique exogène comprenant un gène d'intérêt;
(b) la production de fragments chromosomiques de chromosomes contenus dans les protoplastes recombinés;
(c) la fusion des protoplastes recombinés de (b) avec des protoplastes d'une deuxième espèce végétale pour la production de protoplastes fusionnés, la première et la deuxième espèce végétale pouvant être identiques ou différentes; et
(d) l'identification de protoplastes fusionnés de (c) ou de cellules obtenues à partir des protoplastes fusionnés de (c) qui contiennent des fragments chromosomiques comprenant le gène d'intérêt et présentant des activités chromosomiques normales de plantes.

2. Procédé selon la revendication 1, dans lequel (b) comprend l'irradiation des protoplastes.

3. Procédé selon la revendication 1, dans lequel (b) comprend la mise en contact des protoplastes avec un agent chimique.

4. Procédé selon la revendication 3, dans lequel l'agent chimique est la calichéamicine, l'espéramicine, la dynémicine ou la néocarzinostatine.

5. Procédé selon l'une quelconque des revendications 1 à 4, dans lequel ladite identification de (d) comprend une électrophorèse sur gel à champ pulsé.

6. Procédé selon l'une quelconque des revendications 1 à 5, dans lequel ladite deuxième espèce végétale est la même que ladite première espèce végétale.

7. Procédé selon l'une quelconque des revendications 1 à 5, dans lequel ladite deuxième espèce végétale est un membre de la même famille que ladite première espèce végétale.

8. Procédé selon l'une quelconque des revendications 1 à 7, dans lequel ladite première espèce végétale est une monocotylédone.

9. Procédé selon l'une quelconque des revendications 1 à 7, dans lequel ladite première espèce végétale est une dicotylédone.

10. Procédé selon l'une quelconque des revendications 1 à 9, comprenant en outre (e) la régénération d'une plante entière à partir des protoplastes recombinés de (a), avant (b).

11. Procédé selon l'une quelconque des revendications 1 à 9, comprenant en outre (f) la régénération d'une plante entière à partir des protoplastes fusionnés ou des cellules de plante identifiés dans (d) de la revendication 1.

12. Procédé selon l'une quelconque des revendications 1 à 11, dans lequel l'acide nucléique exogène comprend au moins un site de recombinaison.

13. Procédé selon l'une quelconque des revendications 1 à 12, dans lequel l'acide nucléique exogène comprend au moins un site de restriction.

14. Procédé selon l'une quelconque des revendications 1 à 13, dans lequel l'acide nucléique exogène comprend au moins une région codante.

15. Procédé selon l'une quelconque des revendications 1 à 14, dans lequel l'acide nucléique exogène comprend au moins une séquence comprenant un élément chromosomique de levure.

16. Procédé selon l'une quelconque des revendications 1 à 15, dans lequel l'acide nucléique exogène comprend un chromosome artificiel de levure.

17. Procédé selon l'une quelconque des revendications 1 à 16, dans lequel l'acide nucléique exogène comprend une séquence codant pour un marqueur sélectionnable.

18. Procédé de préparation d'une plante transgénique comprenant
(a) la préparation de protoplastes recombinés d'une première espèce végétale contenant un acide nucléique exogène comprenant un gène d'intérêt;
(b) la production de fragments chromosomiques de chromosomes contenus dans les protoplastes recombinés;
(c) la fusion des protoplastes recombinés de (b) avec des protoplastes d'une deuxième espèce végétale pour la production de protoplastes fusionnés, la première et la deuxième espèce végétale pouvant être identiques ou différentes;
(d) l'identification de protoplastes fusionnés de (c) ou de cellules obtenues à partir des protoplastes fusionnés de (c) qui contiennent des fragments chromosomiques présentant des activités chromosomiques normales de plantes; et
(e) la régénération d'une plante entière à partir des protoplastes ou des cellules identifiés dans (d) qui contiennent lesdits fragments chromosomiques comprenant le gène d'intérêt et présentant des activités chromosomiques normales de plantes.

19. Procédé de fabrication d'un chromosome artificiel de plante, comprenant
(a) la production de plantes transformées d'une première espèce végétale contenant un acide nucléique exogène comprenant un gène d'intérêt;
(b) la production de fragments chromosomiques de chromosomes de ladite première espèce végétale;
(c) le croisement de ladite première espèce végétale contenant les fragments chromosomiques avec une deuxième espèce végétale pour la production d'une espèce végétale hybride, lesdites première et deuxième espèces végétales pouvant être identiques ou différentes; et
(d) l'identification de l'espèce végétale hybride de (c) ou de cellules ou de protoplastes de celle-ci, contenant au moins un fragment chromosomique comprenant le gène d'intérêt et présentant des activités chromosomiques normales de plantes.

20. Cellules végétales ou protoplastes isolés contenant au moins un fragment chromosomique de plante présentant des activités chromosomiques normales produit par le procédé de la revendication 1, le fragment chromosomique comprenant le gène d'intérêt.

21. Culture de protoplastes ou de cellules végétales identifiés dans (d) de la revendication 1.

22. Plante entière produite par le procédé de la revendication 11.

23. Plante entière selon la revendication 22, qui est une plante monocotylédone.

24. Plante entière selon la revendication 22, qui est une plante dicotylédone.

25. Graine provenant de la plante entière selon la revendication 22, la graine comprenant le gène d'intérêt.

26. Plante entière régénérée par le procédé de la revendication 18.

27. Culture de cellules végétales provenant de la plante entière de la revendication 25.

28. Graine provenant de la plante entière de la revendication 26.

29. Espèce végétale hybride ou cellules ou protoplastes de celle-ci, contenant au moins un fragment chromosomique qui présente des fonctions chromosomiques normales de plantes, produit par le procédé de la revendication 19, le fragment chromosomique comprenant le gène d'intérêt.

30. Procédé selon la revendication 15, dans lequel l'élément chromosomique de levure comprend une première séquence centromérique fonctionnelle dans une cellule de levure,

31. Procédé selon la revendication 30, dans lequel les fragments chromosomiques comprennent en outre une deuxième séquence centromérique fonctionnelle dans une cellule végétale.

32. Procédé selon la revendication 17, dans lequel l'acide nucléique exogène comprend en outre au moins un élément chromosomique de levure.

33. Procédé selon la revendication 32, dans lequel l'élément chromosomique de levure comprend une première séquence centromérique fonctionnelle dans une cellule de levure.

34. Procédé selon la revendication 33, dans lequel le fragment chromosomique comprend une deuxième séquence centromérique fonctionnelle dans une cellule végétale.

35. Acide nucléique recombiné comprenant une première séquence centromérique fonctionnelle dans une cellule de levure, et une deuxième séquence centromérique fonctionnelle dans une cellule végétale, isolé à partir des protoplastes fusionnés de (d) dans la revendication 31, ou de (d) dans la revendication 34, ledit acide nucléique recombiné présentant des activités chromosomiques normales de plantes.
